# EUROPEAN PATENT SPECIFICATION

(11) **EP 0 993 822 B1**
(45) Date of publication and mention of the grant of the patent: **04.06.2003**
(21) Application number: 99119072.9
(22) Date of filing: 30.09.1999
(51) Int. Cl.: A61K 7/48

(54) **Ceramide production-accelerating agent**
Ceramid-Produktion Beschleuniger
Agent accélérant la production de céramides

(30) Priority: 30.09.1998 JP 27790398; 28.04.1999 JP 12240299
(43) Date of publication of application: 19.04.2000
(73) Proprietor: Kao Corporation, Tokyo 103-8210 (JP)
(72) Inventor: Ohkubo, Kohji, c/o Kao Corporation, Haga-gun, Tochigi 321-3497 (JP); Takagi, Yutaka, c/o Kao Corporation, Haga-gun, Tochigi 321-3497 (JP); Takatoku, Hiroko, c/o Kao Corporation, Haga-gun, Tochigi 321-3497 (JP); Hori, Kimihiko, c/o Kao Corporation, Haga-gun, Tochigi 321-3497 (JP); Kusuoku, Hiroshi, c/o Kao Corporation, Haga-gun, Tochigi 321-3497 (JP); Shibuya, Yusuke, c/o Kao Corporation, Haga-gun, Tochigi 321-3497 (JP)
(74) Representative: HOFFMANN - EITLE

(56) References cited:
- WO-A-97/06659
- WO-A-98/29093
- GB-A- 2 095 553
- DATABASE WPI Week 9814 Derwent Publications Ltd., London, GB; AN 1998-148351 XP002130654 & JP 10 017459 A (POLA CHEM IND INC)

## Description

### TECHNICAL FIELD

The present invention relates to a ceramide production-accelerating agent which can increase ceramide in the horny layer to restore the barrier function and moisturizing function of the skin.

### BACKGROUND ART

Ceramide which is one of sphingolipids is a lipid contained only in a small amount in the whole living body, but makes up at least half of lipids contained in the horney layer and fulfills an important role in the moisturizing mechanism and barrier mechanism of the skin. The ceramide is produced and secreted in epidermic cells and then functions by forming a lamella structure between cells in the horney layer. However, many reports have been made about the fact that the healthy metabolism of the ceramide is prevented in dermal diseases such as xeroderma, skin roughness, atopic dermatitis, senile xerosis and psoriasis, so that the amount of ceramide in the horney layer is decreased to cause the deterioration of moisturizing function and barrier function of the skin, and the like.

Therefore, a method of supplying the decreased ceramide from the outside is attempted to treat such a dermal disease. However, this method involves such problems that the effect does not last much longer, and stability is low.

It is an object of the present invention to provide a ceramide production-accelerating agent by which the ceramide producing mechanism inherent in the living body can be healthily restored, thereby increasing the amount of ceramide decreased in the horney layer to restore the skin having high barrier function and moisturizing function.

### DISCLOSURE OF THE INVENTION

With the foregoing circumstances in view, the present inventors have carried out an investigation as to the ceramide production-accelerating effects of a great number of plant extracts *in vitro* and *in vivo.* As a result, it has been found that specific plant extracts, which will be described subsequently, have an excellent ceramide production-accelerating effect and can be used in such an application field.

According to the present invention, there is thus provided use of a plant selected from the group consisting of eucalyptus, hop, zingiber, *Uncaria gambir* Roxburgh, *Rosa multiflora* Thunberg, horse chestnut, lily, Job's-tears, cattail, loquat, cape jasmine, *Panax ginseng* C. A. Meyer, *Saponaria officinalis* Linne, white birch, hydrangea, clove, safflower, *Sanguisorba officinalis* Linne, iris and *Sophora flavescens* Aiton, or an extract, steam distilled product or pressed product thereof for the preparation of a ceramide production-accelerating agent.

### BRIEF DESCRIPTION OF THE DRAWINGS

Fig. 1 diagrammatically illustrates the ceramide production-accelerating effects of extracts of eucalyptus (*Eucalyptus globulus),* hop and ginger (zingiber) on human keratinocytes.
Fig. 2 diagrammatically illustrates the effects of increasing the amount of ceramide in the epidermis and horney layer by the extracts of eucalyptus *(Eucalyptus globulus*), hop and ginger (zingiber).
Fig. 3 diagrammatically illustrates the ceramide production-accelerating effects of extracts of gambier (*Uncaria gambir* Roxburgh), rose fruit (*Rosa multiflora* Thunberg), *marronnier* (horse chestnut), lily, *Coicis semen* (Job's-tears), cattail (*Typha angustifolia* linne), loquat, cape jasmine, ginseng (*Panax ginseng* C. A. Meyer), *Saponaria officinalis* Linne, white birch (*Betula pendula* Roth), hydrangea, clove, safflower, *Sanguisorba officinalis* Linne, iris *(Iris florentina* L.) and *Sophora flavescens* Aiton on human keratinocytes.

### MODE FOR CARRYING OUT THE INVENTION

The above plants useful in the practice of the present invention have been known to have various kinds of medicinal effects. However, nothing has been known about their accelerating effect on the production of ceramide.

Among the plants used in the present invention, eucalyptus is a plant of *Eucalyptus globulus or* any other related species thereof, belonging to the family *Myrtaceas,* and its leaves, twigs, blossoms or fruits are mainly used.

Hop (*Humulus lupulus*) is a plant belonging to the family *Moraceae,* and its female flower spikes are mainly used.

Zingiber (*Zingiber officinale* Roscoe) is a plant belonging to the family *Zingiberaceae,* and its rhizome (ginger) is mainly used.

*Uncaria gambir* Roxburgh (gambier) is a plant belonging to the family *Rubiaseae*, and its leaves or young branches are mainly used.

*Rosa multiflora* Thunberg is a plant belonging to the family *Rosaceae,* and its false fruits or fruits (nuts) (i.e., rose fruit) are mainly used.

Horse chestnut (*Aesculus hippocastanum* Linne) is a plant belonging to the family *Hippocastanaceae,* and its seeds, leaves or bark is mainly used.

Lily (*Lilium candidum*) is a plant belonging to the family *Liliaceae,* and its bulb is mainly used.

Job's-tears (*Coix lacryma-jobi* Linne var. *ma-yuen* Stapf) is a plant belonging to the family *Gramineae*), and its seeds (*Coicis semen)* from which a seed coat has been removed are mainly used.

Cattail is a plant of *Typha angustifolia* linne or any other related species thereof, belonging to the family *Typhaceae,* and its flower spikes are mainly used

Loquat (*Eriobotrya japonica* Lindley) is a plant belonging to the family *Rosaceae,* and its leaves are mainly used.

Cape jasmine *(Gardenia jasminoides* Ellis) is a plant belonging to the family *Robiaseae,* and its fruits are mainly used.

*Panax ginseng* C. A. Meyer *(Panax schinseng* Nees) is a plant belonging to the family *Araliaceae,* and its root or a steamed and dried product thereof is mainly used.

*Saponaria officinalis* Linne is a plant belonging to the family *Caryophyllaceae,* and its leaves or root is mainly used.

White birch is a plant of *Betula pendula* Roth or any other related species thereof, belonging to the family *Betulaceae,* and its leaves, bark, xylem or sap is mainly used.

Hydrangea *(Hydrangea serrata* Seringe var. *thunbergii* Sugimoto; *Hydrangea macrophylla* Seringe var. *thunbergii* Makino) is a plant belonging to the family *Saxifragaceae,* and its leaves or the tips of branches thereof are mainly used.

Clove (*Syzygium aromaticum* Merrill et Perry; *Eugenia caryophyllata* Thunberg) is a plant belonging to the family *Myrtaceae*), and its spikes (ears), flower stalks, immature fruits or leaves are mainly used.

Safflower (*Carthamus tinctorius* Linne) is a plant belonging to the family *Compositae,* and its flower, a portion obtained by removing most of a yellow pigment from the flower, or the whole thereof is mainly used.

*Sanguisorba officinalis* Linne is a plant belonging to the family *Rosaceae,* and its root or rhizome is mainly used.

Iris is a plant belonging to the family *Iridaceae,* exemplified by *Iris florentina* L., *Iris germania* L., *Iris pallida* L., etc., and its rhizome is mainly used.

*Sophora flavescens* Aiton is a plant belonging to the family *Leguminosae,* and its root or a portion obtained by removing most of the periderm of the root is mainly used.

In the present invention, the above-described plants may be used as they are, or after they are dried and ground. However, extracts, steam distilled products or pressed products thereof may also be used. More purified products thereof, such as essential oils, may also be used, or marketing products may also be utilized. These plants, or the extracts, steam distilled products or pressed products thereof may be used either singly or in any combination thereof.

Examples of a solvent used in extraction include those routinely used in extraction of plant components, such as water, petroleum ether, n-hexane, toluene, dichloroethane, chloroform, ether, ethyl acetate, acetone, methanol, ethanol, propanol, butanol, ethylene glycol, propylene glycol and butylene glycol. Of these, water, ethanol, propylene glycol and butylene glycol are particularly preferred. These solvents may be used either singly or in any combination thereof. Ordinary conditions may be likewise applied to conditions for the extraction. For example, it is only necessary to immerse any one of the above-described plants at 3 to 100°C for several hours to several weeks in the solvent or heat it under reflux. Even when the plant is used as an essential oil, the conventional method may be adopted. For example, the essential oil may be obtained from any one of the above-described plants in accordance with the steam distillation method, extraction method, pressing method or the like. The extracts, steam distilled products or pressed products of these plants may be used as active ingredients for a ceramide production-accelerating agent according to the present invention as they are. However, a fraction high in activity may be fractionated by a proper isolating means, for example, gel filtration, chromatography, rectification or the like to use it.

The ceramide production-accelerating agent according to the present invention is low in toxicity and may be administered in either method of external application or internal use. Examples of an object into which the ceramide production-accelerating agent according to the present invention is incorporated include ointments, lotions, creams, beauty lotions, cosmetic lotions, massaging compositions, packs, foundations, lipsticks, bath additive compositions, shampoos, hair conditioners, hair tonics, tablets and capsules. No particular limitation is imposed on other additives incorporated into these products, and publicly known bases may be used. No particular limitation is imposed on the amount of the ceramide production-accelerating agent according to the present invention to be incorporated into these products. However, it is preferably incorporated in a range of about 0.00001 to 20 % by weight, particularly 0.0001 to 10 % by weight in terms of solid content.

### EXAMPLES

### Preparation Example 1: Preparation of eucalyptus extract

Leaves of *Eucalyptus globulus* Labillardiere were cut into pieces, and a mixed solvent (20:80; 100 ml) of water and 1,3-butanediol was added to the cut pieces (10 g) to conduct extraction at room temperature for 24 hours while sometimes stirring the mixture. The resultant extract was then filtered, and the filtrate was left at rest for 7 days at 5°C to age the filtrate, and dregs and precipitate formed were separated by filtration. Water (100 ml) was added to the resultant filtrate, and the mixture was concentrated to about 70 ml at 40°C under reduced pressure. After this process was repeated 3 times, water and 1,3-butanediol were added in such a manner that the concentration of 1,3-butanediol was adjusted to 80 v/v%, and the whole solution amounted to 100 ml.

### Preparation Example 2:

Various kinds of crude drug extracts (each 100 ml) shown in Table 1 were prepared in accordance with a method known *per se* in the art.

**Table 1**

| Crude drug extract | Plant | Extraction solvent |
|---|---|---|
| Hop extract | Female spikes of *Humulus lupulus* | 1,3-Butanediol |
| Ginger extract | Rhizone of *Zingiber officinale* Roscoe | Water:ethanol = 50:50 |
| Gambier extract | Leaves or young branches of *Uncaria gambir* Roxburgh | Water→ water:ethanol = 50:50 |
| Rose fruit extract | Fruits of *Rosa multiflora* Thunberg | Water:ethanol = 50:50 |
| *Marronnier* extract | Seeds of *Aesculus hippocastanum* Linne | Water:1,3-butanediol = 50:50 |
| Lily extract | Bulb of *Linium candidum*) | Water:1,3-butanediol = 50:50 |
| *Coicis semen* extract | Seeds of *Coix lacryma-jobi* Linne var. *ma-yuen* Stapf from which a seed coat has been removed | Water:1,3-butanediol = 50:50 |
| Cattail extract | Flower spikes of *Typha angustifolia* linne | Water:1,3-butanediol = 10:90 |
| Loquat leaf extract | Leaves of *Eriobotrya japonica* Lindley | Water:1,3-butanediol = 10:90 |
| Cape jasmine | Fruits of *Gardenia jasminoides* Ellis | 1,3-Butanediol |
| Ginseng extract | Root of *Panax ginseng* C. A. Meyer | Water:ethanol = 50:50 |
| *Saponaria officinalis* Linne | Leaves of *Saponaria officinalis* Linne | Water:1,3-butanediol = 50:50 |
| White birch extract | Bark or xylem of *Betula pendula* Roth | Water:ethanol = 50:50 |
| Hydrangea extract | Leaves or branch tips of *Hydrangea serrata* Seringe var. *thunbergii* Sugimoto | Water:ethanol = 50:50 |
| Clove extract | Spikes (ears) of *Syzygium aromaticum* Merrill et Perry | Water:ethanol = 50:50 |
| Safflower extract | The whole *Carthamus tinctorius Linne* | Water:ethanol = 50:50 |
| *Sanguisorba officinalis* Linne extract | Root or rhizome or *Sanguisorba officinalis* Linne | Water:ethanol = 50:50 |
| Iris root extract | Rhizome of *Iris florentina* L. | Ethanol |
| *Sophora flavescens* Aiton extract | Root of *Sophora flavescens* Aiton | Water:ethanol:1,3-butanediol = 50:30:20 |

### Example 1: Test for acceleration of ceramide production (cell system)

### <Method>

Human keratinocytes (HK-f; product of Kyokuto Seiyaku Kogyo K.K.) were cultured for 24 hours at 37°C under 5% CO₂ in a medium (GIBCO SFM/-BPE, EGF) containing [¹⁴C]-serine (product of Daiichi Pure Chemicals Co., Ltd.) using a 6-well plate. The crude drug extract of eucalyptus, hop or ginger obtained in Preparation Example 1 or 2 was then added to the medium in a proportion of 0.001 % by weight or 0.01 % by weight in terms of solid content to conduct the culture for additional 24 hours. After the medium was removed, and the wells were washed once with PBS, cells were scraped with a cell scraper to collect them in a test tube. After water (3.6 ml), chloroform (4 ml) and methanol (4 ml) were added to the human keratinocytes in this test tube to mix them, a chloroform layer was isolated and dried to solid. The lipid extracted was developed to the top twice with a solvent 1 (chloroform:methanol:acetic acid = 190:9:1) and to 3 cm from the bottom with a solvent 2 (chloroform:methanol: acetone = 76:20:4) on a HPTLC plate [silica gel G60 (20 x 10 cm), Art.5641; product of Merck Co.]. The counts of ceramide and glycosylceramide on the TLC plate were measured by means of an autoradiograph (BAS2000; manufactured by Fuji Photo Film Co., Ltd.).

### <Results>

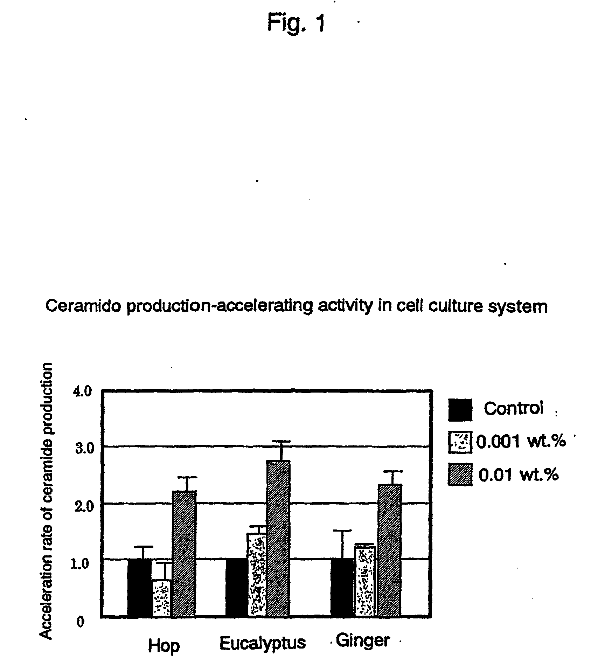

The results obtained by calculating out an acceleration rate of ceramide production with the acceleration rate of a control, to which no crude drug extract was added, regarded as 1.0 are shown in Fig. 1. As apparent from Fig. 1, all extracts of eucalyptus, hop and ginger were observed having a ceramide production-accelerating effect on human keratinocytes.

### Example 2: Test for acceleration of ceramide production (animal system)

### <Method>

The plant extract (0.1 % by weight, 0.01 % by weight or 0.001 % by weight in terms of solid content) of eucalyptus, hop or ginger obtained in Preparation Example 1 or 2, which had been diluted with a 7:3 mixed solvent of propylene glycol and ethanol, was applied to the back of a hairless mouse for 2 weeks, and the skin was then cut out of the back. The skin was subjected to a heat treatment at 60°C for 60 seconds, thereby peeling the epidermis from the skin. The epidermis was divided into halves, and the horney layer was prepared from one of them using 0.5% trypsin. After the epidermis and horney layer were lyophilized, and their weights were measured, lipid extraction was conducted in accordance with the Bligh/Dyer method (chloroform:methanol:water = 4:4:3.6), and the extracts were subjected to HPTLC in the same manner as in Example 1. After development, the plate was immersed in a solution containing 8 % by weight of phosphoric acid and 10 % by weight of copper sulfate to conduct printing at 160°C for 15 minutes, and ceramide was then determined by means of a densitometer (Bio·Image; manufactured by Bio·Image Co.).

### <Results>

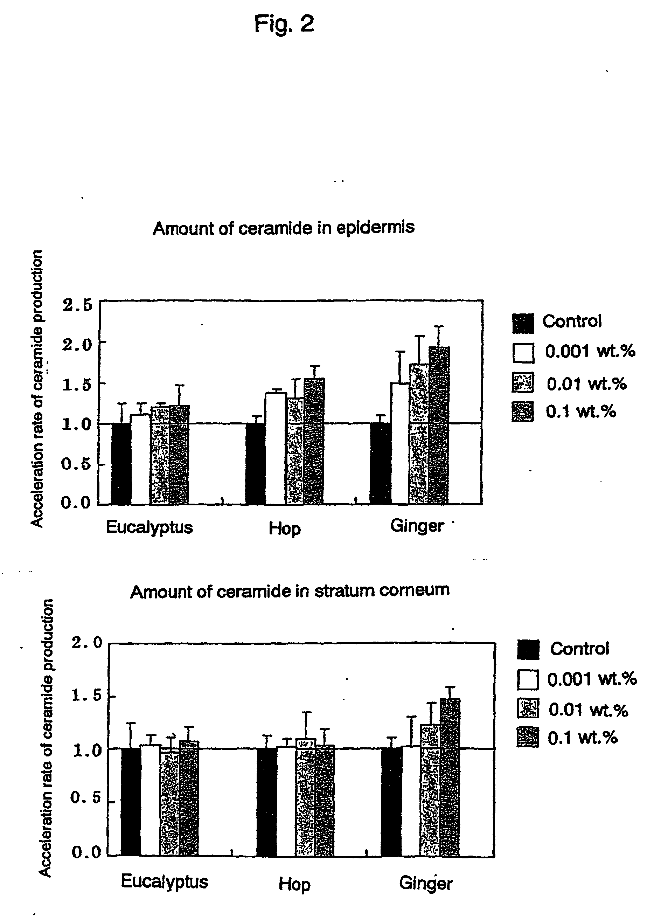

The results obtained by calculating out a quantitative proportion of ceramide with the amount of ceramide in a control, to which no crude drug extract was added, regarded as 1.0 are shown in Fig. 2. As apparent from Fig. 2, all extracts of eucalyptus, hop and ginger were observed having an effect of increasing the amounts of ceramide in the epidermis and horney layer.

### Example 3: Test for acceleration of ceramide production (cell system)

### <Method>

A test for acceleration of ceramide production was conducted in the same manner as in Example 1 except that the extracts of gambier, rose fruit, *marronnie,* lily, *Coicis semen*, cattail, loquat, cape jasmine, ginseng. *Saponaria officinalis* Linne, white birch, hydrangea, clove, safflower, *Sanguisorba officinalis* Linne, iris root and *Sophora flavescens* Aiton obtained in Preparation Example 2 were separately used.

### <Results>

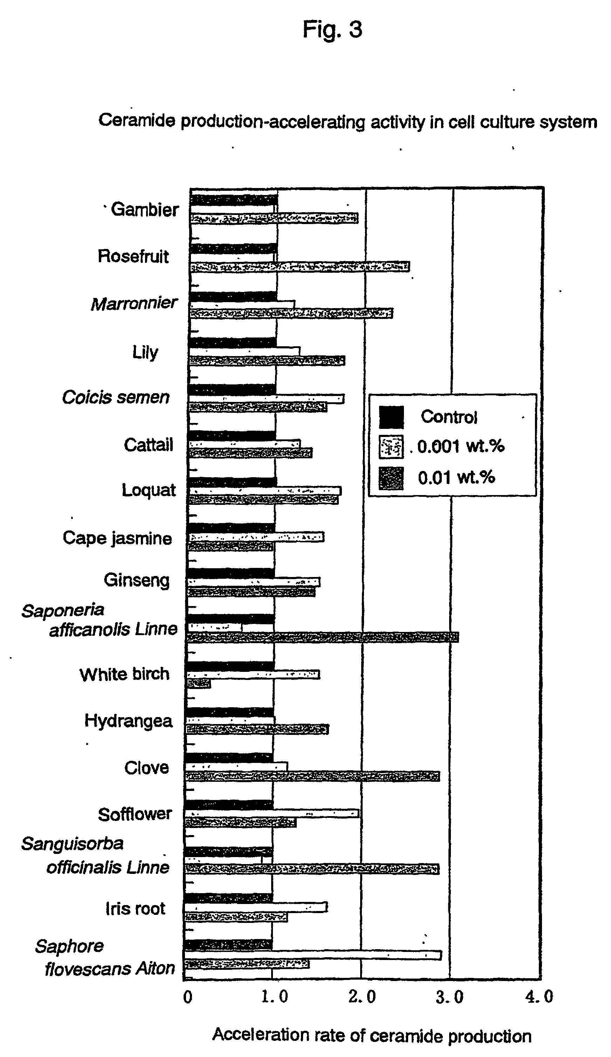

The results obtained by calculating out an acceleration rate of ceramide production with the acceleration rate of a control, to which no crude drug extract was added, regarded as 1.0 are shown in Fig. 3. As apparent from Fig. 3, all the extracts were observed having a ceramide production-accelerating effect on human keratinocytes.

### INDUSTRIAL APPLICABILITY

The ceramide production-accelerating agents according to the present invention can healthily restore the ceramide producing mechanism inherent in the living body, thereby increasing the amount of ceramide decreased in the horney layer to restore the barrier function and moisturizing function of the skin.

## Claims

1. Use of a plant selected from the group consisting of eucalyptus, hop, zingiber, *Uncaria gambir* Roxburgh, *Rosa multiflora* Thunberg, horse chestnut, lily, Job's-tears, cattail, loquat, cape jasmine, *Panax ginseng* C. A. Meyer, *Saponaria officinalis* Linne, white birch, hydrangea, clove, safflower, *Sanguisorba officinalis* Linne, iris and *Sophora flavescens* Aiton, or an extract, steam distilled product or pressed product thereof for the preparation of a ceramide production-accelerating agent.

2. The use according to Claim 1, wherein eucalyptus, or the extract, steam distilled product or pressed product thereof is used.

## Patentansprüche

1. Verwendung einer Pflanze, ausgewählt aus der Gruppe, bestehend aus Eukalyptus, Hopfen, Zingiber, Uncaria gambir Roxburgh, Rosa multiflora Thunberg, Rosskastanien, Lilie, Tränengras, Rohrkolben, Loquat, Cape Jasmin, Panax ginseng C. A. Meyer, Saponaria officinalis Linne, Weissbirke, Hydrangea, Gewürznelke, Safflor, Sanguisorba officinalis Linne, Iris und Sophora flavescens Aiton, oder eines Extraktes, dampfdestillierten Produktes oder gepressten Produktes davon zur Erzeugung eines Mittels zu Beschleunigung der Ceramid-Produktion.

2. Verwendung nach Anspruch 1, worin Eukalyptus oder der Extrakt, das dampfdestillierte Produkt oder das gepresste Produkt davon verwendet wird.

## Revendications

1. Utilisation d'une plante choisie parmi le groupe constitué de l'eucalyptus, du houblon, du gingembre, de *Uncaria gambir* Roxburgh, de *Rosa multiflora Thunberg,* du marron d'Inde, du lis, de la larme de Job, de la quenouille, de la nèfle du Japon, du jasmin du Cap, de *Panax ginseng* C. A. Meyer, de *Saponaria officinalis* Linne, du bouleau blanc, de l'hortensia, du clou de girofle, du carthame, de *Sanguisorba officinalis* Linne, de l'iris et de *Sophora flavescens* Aiton, ou d'un extrait, d'un produit distillé à la vapeur ou d'un produit pressé de ceux-ci en vue de la préparation d'un agent d'accélération de la production de céramides.

2. Utilisation selon la revendication 1, dans laquelle l'eucalyptus, ou l'extrait, le produit distillé à la vapeur ou le produit pressé de celui-ci est utilisé.
